# EUROPEAN PATENT APPLICATION

(11) **EP 2 025 297 A2**
(43) Date of publication of application: **18.02.2009**
(21) Application number: 08009109.3
(22) Date of filing: 16.05.2008
(51) Int. Cl.: A61B 18/12

(54) **Electric treatment system**

(30) Priority: 14.08.2007 US 838380
(71) Applicant: Olympus Medical Systems Corp., Tokyo 151-0072 (JP)
(72) Inventor: Mihori, Takashi, Hachioji-shi Tokyo 192-8512 (JP); Irisawa, Takashi, Hachioji-shi Tokyo 192-8512 (JP); Tanaka, Kazue, Hachioji-shi Tokyo 192-8512 (JP); Shirai, Tamaki, Hachioji-shi Tokyo 192-8512 (JP); Kabaya, Akinori, Hachioji-shi Tokyo 192-8512 (JP)
(74) Representative: von Hellfeld, Axel

(57) **Abstract**

An electric treatment system is provided with a treatment instrument which subjects an objective living tissue (10) to a first treatment based on a cauterization process for adhesion using high-frequency power and a second treatment based on a desiccation process for dehydrating a region healed by the adhesion, a detector for detecting a phase difference signal from the output high-frequency power, and a controller which controls the entire system. The controller switches the first treatment over to the second treatment when the phase difference signal is detected. When a predetermined phase difference as a criterion is exceeded by the phase difference detected during the second treatment, the controller stops the output of the high-frequency power, whereupon the second treatment terminates.

## Description

The present invention relates to an electric treatment system for a surgical treatment, such as blood vessel sealing for coagulation or hemostasis of a living tissue using high-frequency power.

There are generally known electric operation apparatuses, e.g., electric knives, which use high-frequency power for a surgical treatment, such as incision, coagulation, or hemostasis, for living tissues. The electric operation apparatuses can be classified into two types, monopolar and bipolar, according to high-frequency power application mode and the configuration of treatment instruments for treating living tissues. A bipolar treatment instrument is in the form of a forceps that has two distal end portions electrically isolable from each other and serves to seal and weld blood vessels or vascular bundles. In sealing and welding a blood vessel or a part of a body cavity of a living tissue, the extent of desiccation or the state of dehydration of the tissue is feedback-controlled by monitoring the impedance of the tissue. In order to enhance a sealing force for the blood vessel or the like, in particular, degeneration of medium intima and dehydration are essential. Since a process for the dehydration must be performed in consideration of heat to be applied to the living tissue, a technique for intermittently applying switched high-frequency power in accordance with detected impedance is proposed in Jpn. Pat. Appln. KOKAI Publication No. 10-225462, for example.

The object of the present invention is to provide an electric treatment system and a treatment method in which a high-frequency power application mode and switching and termination of a process are determined based on a phase difference generated in applied high-frequency power, and appropriate cauterization and desiccation of a living tissue to be treated can be realized without regard to variation in the composition and water content of the tissue.

According to an aspect of the invention, there is provided an electric treatment system comprising a treatment instrument which performs for a living tissue a first treatment based on a cauterization process for adhesion using high-frequency power, and a second treatment based on a desiccation process for dehydrating a region healed by the adhesion, a cable through which the high-frequency power is transmitted to the treatment instrument, an output section which is provided with a first output control mode in which the high-frequency power is continuously output to perform the first treatment, and a second output control mode in which the high-frequency power is intermittently supplied to perform the second treatment at intervals based on predetermined output stop times, and alternatively outputs the high-frequency power in the first or second output control mode, a phase difference detector which detects, as a phase difference signal, a phase difference generated between a high-frequency voltage and a high-frequency current output from the output section, based on a combined capacitance between the treatment instrument and the cable, and a controller which controls the entire system, switches the first output control mode over to the second output control mode as the first treatment is replaced by the second treatment when the phase difference signal is detected from the phase difference detector, and stops the output of the high-frequency power when a predetermined phase difference signal as a criterion is exceeded by the phase difference signal detected in the second output control mode.

According to another aspect of the invention, there is provided a treatment method using an electric treatment system, comprising a first treatment based on a cauterization process for adhesion in which continuously output high-frequency power is applied to an objective living tissue held by a treatment instrument, and a second treatment based on a desiccation process in which high-frequency power is intermittently applied to the held objective living tissue at intervals based on predetermined output stop times, whereby a region healed by the adhesion is dehydrated, the first treatment being switched over to the second treatment when a phase difference composed of a current component and a voltage component of the high-frequency power applied to the objective tissue is detected. In this treatment method, the output of the high-frequency power is stopped when a predetermined phase difference as a criterion is exceeded by the phase difference composed of the current and voltage components of the high-frequency power applied to the objective tissue.

The invention can be more fully understood from the following detailed description when taken in conjunction with the accompanying drawings, in which:
FIG. 1 is a diagram showing a configuration example of an electric treatment system according to a first embodiment;
FIG. 2 is a diagram showing output impedance vectors viewed from the power source side;
FIG. 3 is a flowchart for illustrating a treatment process in the electric treatment system according to the first embodiment;
FIG. 4 is a high-frequency power waveform diagram for illustrating a judgment for the transition from a cauterization process to a desiccation process;
FIG. 5 is a high-frequency power waveform diagram for illustrating a judgment for the termination of the desiccation process;
FIG. 6 is a view showing an example of a holding structure of a treatment instrument used in the first embodiment;
FIG. 7 is a view showing a holding structure of a first example of the bipolar treatment instrument used in the first embodiment;
FIG. 8 is a diagram showing a characteristic of a phase difference generated by the treatment instrument of the first example;
FIG. 9 is a view showing a holding structure of a second example of the bipolar treatment instrument used in the first embodiment;
FIG. 10 is a diagram showing a characteristic of a phase difference generated by the treatment instrument of the second example;
FIG. 11 is a diagram showing a configuration example of an electric treatment system according to a second embodiment;
FIG. 12 is a diagram showing a configuration example of an electric treatment system according to a third embodiment;
FIG. 13 is a diagram showing a conceptual configuration of a modification of the third embodiment; and
FIG. 14 shows an example of an identification table used in the modification of the third embodiment.

Embodiments of the present invention will now be described in detail with reference to the accompanying drawings.

The concept of an electric treatment system of the invention will be described first.

In the electric treatment system, high-frequency current is supplied to a bipolar treatment instrument (forceps or the like), whereby a living tissue (hereinafter referred to as the objective tissue) 10 to be treated is incised and coagulated (or cauterized and desiccated). Based on the spirit of the invention, position control is used to discriminate switching of a treatment state and an end of a treatment. In the position control, the timing for switching from a cauterization process for fully degenerating the objective tissue 10, e.g., a medium-inner membrane layer of a blood vessel, to a desiccation (or dehydration) process is determined based on a phase difference between current and voltage in high-frequency power output (specifically, a phase difference between pure resistance and reactance components). Further, a dehydrated state is detected, and a suitable timing for finishing the desiccation process is determined, whereupon the desiccation process is finished.

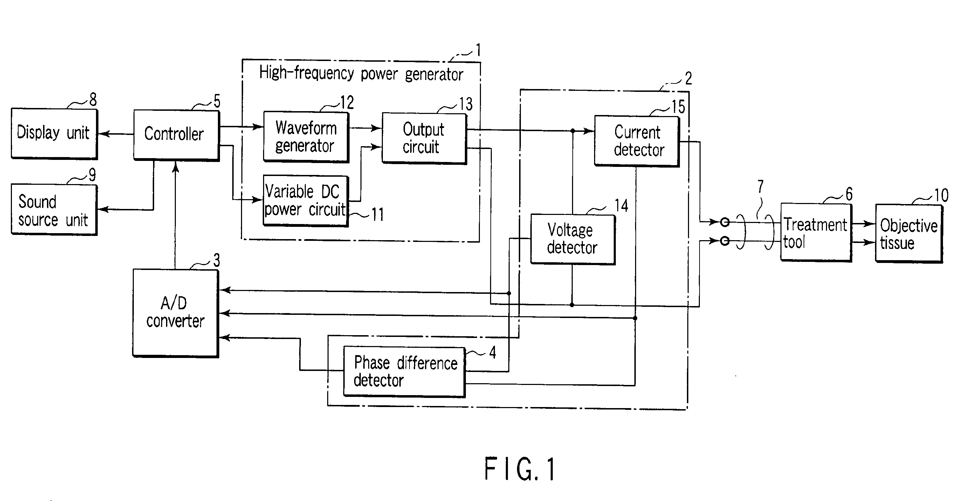

FIG. 1 shows a configuration example of a high-frequency cauterization power source unit and a bipolar treatment instrument (grasping forceps), which constitute an electric treatment system according to a first embodiment. This example will be described in detail.

The electric treatment system generally includes a high-frequency power generator 1, detector 2, A/D converter 3, controller 5, treatment instrument 6, cables 7, and display unit 8 or sound source unit 9. The power generator 1 generates high-frequency power. The detector 2 detects a current signal (current component), voltage signal (voltage component), and phase difference signal from the high-frequency power output. The A/D converter 3 digitizes the detected current, voltage, and phase difference signals. The controller 5 performs parameter calculation (mentioned later) and control of the entire system. The treatment instrument 6 applies high-frequency power for treatment to the objective tissue 10. The cables 7 electrically connect the treatment instrument 6 and the power generator 1 and transmit high-frequency power. The display unit 8 displays input instructions, system state, and treatment (process) conditions. The sound source unit 9 notifies an operator with a buzzer sound (including a chime sound) or a voice.

The high-frequency power generator 1 is composed of a variable DC power circuit 11, a waveform generator 12, and an output circuit 13. The power circuit 11 supplies a DC voltage based on set value information, which will be described later. The waveform generator 12 generates a drive waveform. The output circuit 13 generates and outputs high-frequency power based on the DC voltage and the drive waveform.

The detector 2 is composed of a voltage detector 14, a current detector 15, and a phase difference detector 4. The voltage detector 14 detects a voltage output from the high-frequency power generator 1. The current detector 15 detects a current output from the generator 1. The phase difference detector 4 detects a time difference (phase difference) between the output voltage and current from the generator 1.

The controller 5 calculates an impedance value and an output power value associated with the high-frequency power, as output parameters, based on the voltage and current signals delivered from the A/D converter 3. Further, the controller 5 calculates output parameters of output voltage values, output current values, and phase difference amounts, based on the voltage, current, and phase difference signals, respectively. The controller 5 compares these output parameters with predetermined values and controls the output of the high-frequency power based on results of the comparison. In the present embodiment, the current value is limited by hardware such as a circuit. The parameters other than the current value are compared and controlled by the controller 5.

The high-frequency power generator 1 is provided with at least two modes, a first output control mode (constant-power application mode) and a second output control mode (intermittent-power application mode). The constant-power application mode is suited for the cauterization process in which a high-frequency output is continuously supplied to the objective tissue 10. The intermittent-power application mode is suited for the desiccation process in which high-frequency power is intermittently supplied to the tissue 10 at intervals based on predetermined output stop times. The constant-power application mode is a mode in which a high-frequency output based on a normally constant voltage value is applied. Naturally, in these modes, the voltage and current values of the high-frequency power can be suitably set to desired values by instructing the controller 5. Likewise, the output stop times in the intermittent-power application mode can be set to desired times. Further, a total time during which the high-frequency power is applied can also be set as an application limit time for protecting the objective tissue 10. If the limit time is exceeded, moreover, an error is concluded to have occurred without regard to the treatment state, and the supply of the high-frequency power is stopped at once.

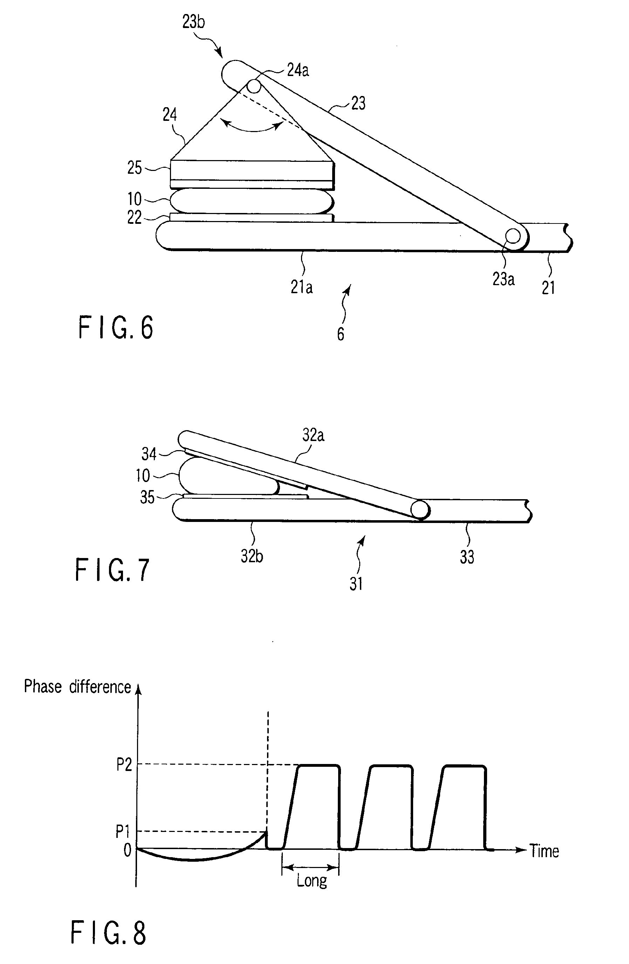

FIG. 6 shows a holding structure of the treatment instrument 6 used in the present embodiment.

In this treatment instrument 6, a sheath 21 is provided with a jaw 21a that extends integrally in a straight line from it. Located on the jaw 21a is a first electrode 22 having a shape such that it can easily hold the objective tissue 10, e.g., the shape of a flat plate in this case. A jaw 23 is mounted in a manner such that its one end (support end portion) 23a is rockably supported on the jaw 21a. The jaw 23 is in the form of a straight bar, of which a distal end portion 23b is located somewhat short of the distal end of the jaw 21a when it is closed. A support portion 24 that is swingably supported by a pivot 24a is provided on the distal end side of the jaw 23 that faces the first electrode 22. The support portion 24 supports a second electrode 25 that has the same shape (shape of a flat plate having a similar opposite surface) as the first electrode 22 in a manner such that the two electrodes face each other. The first and second electrodes 22 and 25 have, for example, a rectangular shape such as an oblong or square. Depending on the site of use, moreover, the first and second electrodes 22 and 25 may be formed having the shape of a circle, ellipse, or track. According to the present embodiment, furthermore, the objective living tissue is held by an electrode pair formed of two parallel flat plates. If the objective living tissue has a peculiar shape, however, the electrode pair may have opposite surfaces that fit the shape of the tissue or the shape of a treated coagulation (desired shape after desiccation).

When the treatment instrument 6 with this construction holds the objective tissue 10 having a circular cross section, e.g., a blood vessel, the flat second electrode 25 extends parallel to the first electrode 22 and can evenly crush the tissue 10. Thus, the cauterization and desiccation processes for the tissue 10 can be advanced uniformly.

The following is a description of the position control in the electric treatment system.

The bipolar treatment instrument 6 for living tissue treatment and the system body (high-frequency cauterization power source unit) are connected to each other by the cables 7. In consideration of actual usage, the cables 7 are given a length of about 3m, for example. Many bipolar instruments use at least two-core cable conductors that connect leads of outward and return paths for high-frequency current. If the outward and return paths for high-frequency current are thus long and located close to each other, a combined capacitance exists between the cable conductors that are coated for insulation. If the impedance of the objective tissue 10 increases when high-frequency power is applied to it, in particular, some of the high-frequency current returns through the combined capacitance between the cables to the power source side and is lost as a leakage current without being applied to the treatment instrument. In the present embodiment, the influence of the combined capacitance component between the cables from the treatment instrument and the system body (high-frequency output circuit) is reduced.

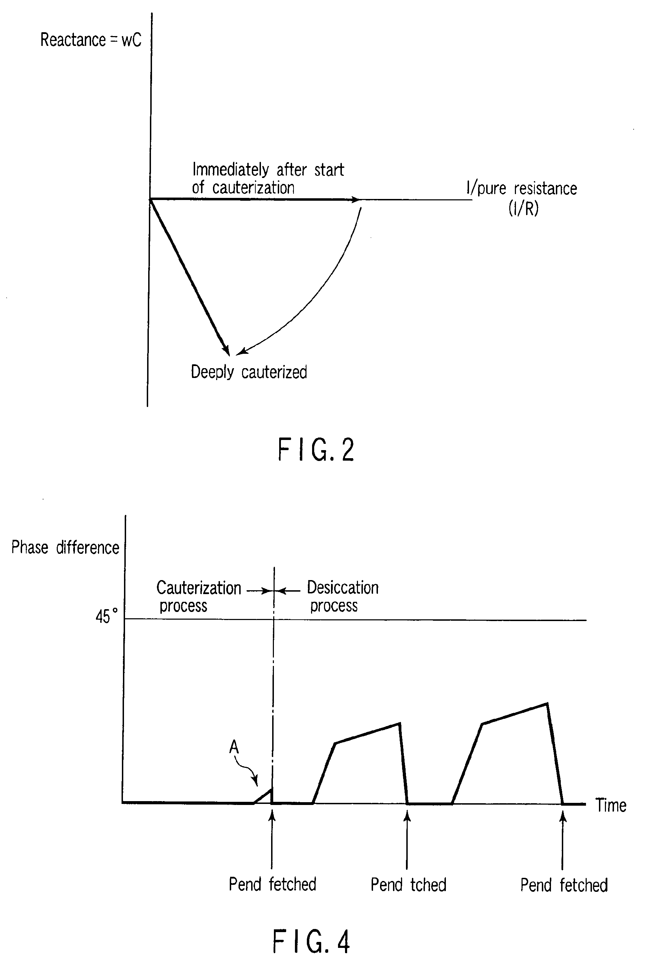

FIG. 2 shows output impedance vectors viewed from the power source side, partially based on the capacitance component between the cables. When viewed from the treatment instrument side, the resistance of the objective tissue 10 and the combined capacitance between the cables are connected in parallel. In FIG. 2, therefore, the abscissa axis represents the reciprocal of the pure resistance, and the ordinate axis represents the reciprocal of the reactance.

Before the cauterization of the objective tissue 10 advances, sufficient water exists in the tissue, so that the resistance is very low. Therefore, the abscissa-axis component (reciprocal of the resistance) in FIG. 2 is a large value. In this case, the reciprocal (eigen value) of the reactance based on the combined capacitance is sufficiently higher than the pure resistance component, and its reciprocal is small. Thus, a phase difference, that is, an angle between the abscissa axis and a resultant vector that combines the respective reciprocals of the pure resistance and the reactance, is very small.

When the cauterization of the objective tissue 10 advances so that dehydration of the tissue is accelerated to increase the resistance, thereafter, the reciprocal of the pure resistance gradually decreases. As mentioned before, the reciprocal of the reactance based on the combined capacitance is an eigen value. As the cauterization process advances to promote dehydration of the tissue, therefore, the resultant impedance vector that combines the respective reciprocals of the pure resistance and the reactance gradually moves toward the ordinate axis, as shown in FIG. 2. In other words, a phase difference or an angle between the resultant impedance vector and the abscissa axis gradually approaches 90 degrees. If the tissue and the electrodes are entirely kept from contacting one another, the phase difference theoretically fully reaches 90 degrees. At this point of time, no high-frequency energy is supplied to the objective tissue 10. Actually, the high-frequency power cannot be applied to the extent of this phase difference.

Thus, the reactance component primarily exhibits a level sufficiently higher than the impedance before cauterization, thus can be used as an index showing advance of cauterization of the tissue.

The objective tissue 10 should not be cauterized and dehydrated excessively, and an energy loss in the applied high-frequency power should be reduced. According to the present embodiment, from this point of view, it is proposed to stop output when the phase difference is approximately 45 degrees as a guideline timing for the completion of a dehydration process. In FIG. 2, the phase difference of 45 degrees indicates equivalence in the respective values of the pure resistance and reactance components. Thus, when the phase difference becomes 45 degrees, the energy that is actually supplied to the tissue is theoretically halved. In other words, half of the total output energy from the power source is transmitted to the tissue, while the remainder is fed back to the high-frequency power source through the combined capacitance between the cables. Empirically, the influence of the application of the high-frequency power to the objective tissue 10 is reduced. Primarily, it is necessary only that the states of cauterization and dehydration of the treated tissue 10 be observed. However, this observation cannot be performed if the object of treatment is a medium-inner membrane layer of a blood vessel of a human body. Since the medium-inner membrane layer is subject to individual differences, it is difficult to settle a quantitative reference. According to the present embodiment, therefore, the phase difference is set to 45 degrees as a criterion obtained by simulation or experiment.

Based on this setting, it is concluded that the desiccation (or dehydration) process for the objective living tissue is completed when a predetermined value (e.g., a set signal value based on the phase difference of 45 degrees) is exceeded by a phase difference signal (mentioned later) detected by the phase difference detector during the desiccation process. Thereupon, the supply of the high-frequency power in the intermittent-power application mode is finished.

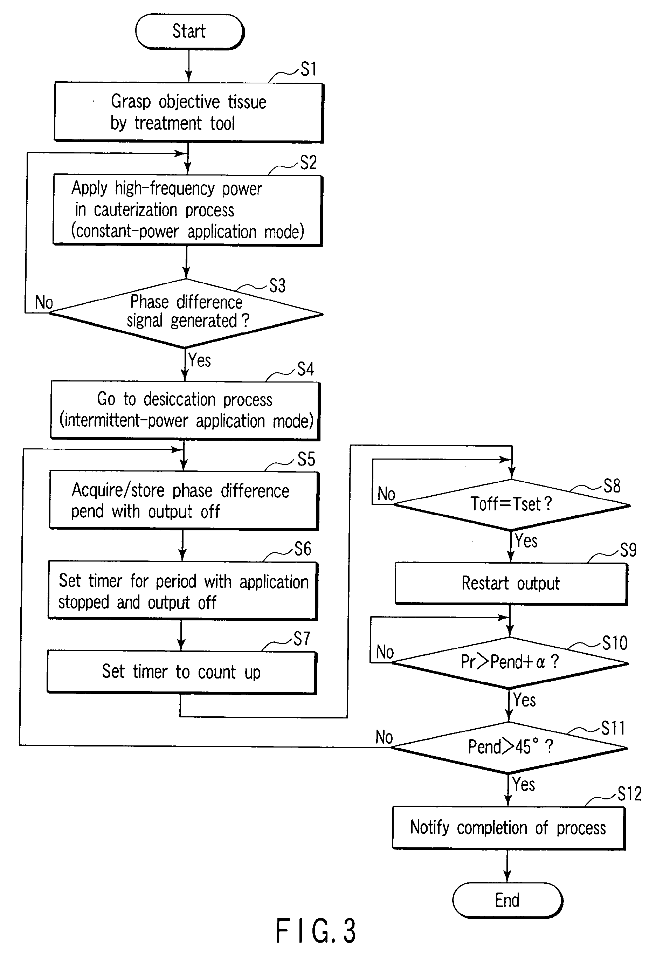

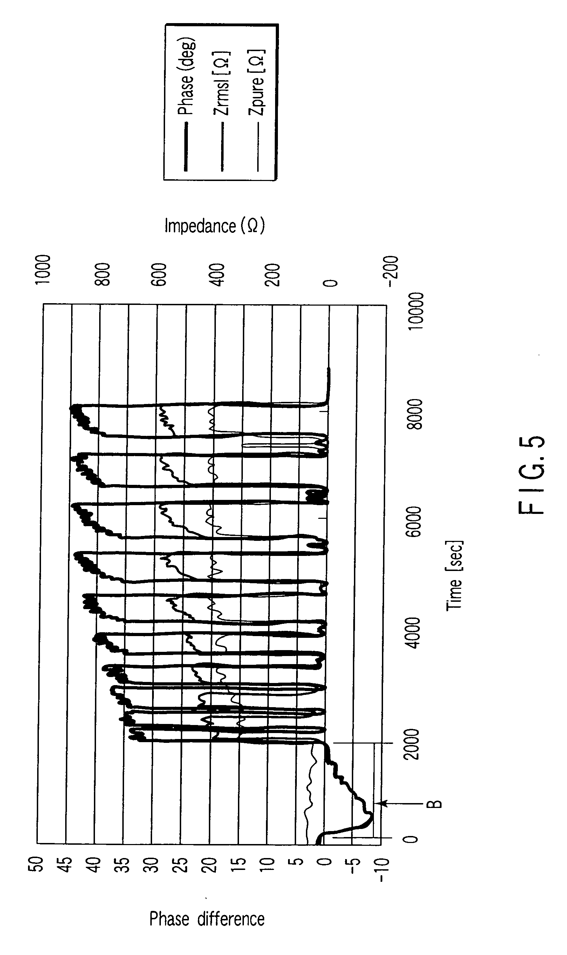

A treatment process in the electric treatment system according to the present embodiment will now be described with reference to the flowchart of FIG. 3 and the high-frequency power waveform diagrams of FIGS. 4 and 5. FIG. 4 is a diagram for illustrating a judgment for the transition from the cauterization process to the desiccation process. FIG. 5 is a diagram for illustrating a judgment for the termination of the desiccation process. In this case, a treatment for sealing the medium-inner membrane layer of the blood vessel as the objective tissue is given by way of example, and the phase difference of 45 degrees is set as a criterion for the termination of the desiccation process.

First, the objective tissue 10 is held by the treatment instrument 6 shown in FIG. 6 (Step S1). A high-frequency output is continuously applied to the instrument 6 in the constant-power application mode, and the cauterization process is performed for the held tissue 10 (Step S2). At this point of time, water initially remains in the tissue 10 in the section of the cauterization process shown in FIG. 4, so that the resistance is very low, as in the aforementioned case. Thus, the phase difference can be kept very small. When the cauterization of the objective tissue 10 advances so that the dehydration of the tissue is accelerated to increase the resistance, thereafter, the resultant impedance vector that combines the respective reciprocals of the pure resistance and the reactance approaches the ordinate axis, as shown in FIG. 2. More specifically, a phase difference signal A is generated, as indicated by the arrow in FIG. 4. It is determined whether or not the signal A is generated (Step S3). For this determination, a change equal to, for example, 10% or more of the voltage value of the high-frequency output may be previously set as a criterion for the process switching. If the phase difference signal A is generated (YES), the cauterization process is then switched over to the desiccation process (Step S4). The value equal to 10% of the voltage value of the high-frequency output in the constant-power application mode, which is a criterion for the process switching, is not an absolute value but an index. Usually, an operator (doctor) visually recognizes numerical information and an image of the objective tissue displayed on the display unit and makes an empirical decision. Therefore, the criterion may be suitably set in consideration of the individual difference of objective tissues and the specific properties of the treatment instrument.

Then, the phase difference signal A at its leading edge is acquired as an output-off phase difference Pend and stored into a memory in the controller 5 (Step S5). This memory may be provided in advance with a table for storing the output-off phase difference Pend so that the value can be updated every time a new signal is acquired.

Then, the output (application) of the high-frequency power to the objective tissue 10 is stopped the moment the phase difference signal A falls. When the application is stopped, an output stop timer in the controller 5 starts counting (Step S6). This output stop timer continues counting until a preset time Tset is reached. The preset time Tset, that is, a time Toff for the continuation of output stop, can be suitably set within a range such that the temperature of the heated tissue 10 never lowers. If the temperature of the tissue 10 lowers while the output is off, the desiccation (or dehydration) is inefficient and additionally time-consuming. Therefore, the preset time should be set carefully.

Subsequently, the output stop timer counts up (Step S7) and determines whether or not the preset time Tset is reached by the stop time Toff (Step S8). The count-up is continued so that it is concluded by this determination that the preset time Tset is reached by the stop time Toff (NO). When the preset time Tset is reached by the stop time Toff (YES), on the other hand, the controller 5 is instructed to start outputting the high-frequency power again to the objective tissue 10 (Step S9).

Then, as shown in FIG. 4, it is determined whether or not a present phase difference Pr (e.g., P3) is larger than the last phase difference Pend (e.g., P3) (Pr > Pend + α), during the output of the high-frequency power (Step S10). Here α is a coefficient, which is set in advance, depending on the shape and holding area of the forceps and variations in the objective tissue 10 (e.g., difference in the amount of water before treatment). If it is concluded by this determination that the phase difference Pr is larger than Pend + α (YES), the phase difference Pr is regarded as an up-to-date phase difference Pend. The phase difference Pend is compared with the predetermined phase difference of 45 degrees (Step S11). If it is then concluded that the phase difference of 45 degrees is not exceeded by the up-to-date phase difference Pend (NO), the program returns to Step S5. After the next output stop time is then cleared, high-frequency power is applied. If the phase difference of 45 degrees is reached by the up-to-date phase difference Pend (YES), on the other hand, the application of the high-frequency power is stopped to terminate the desiccation process, as shown in FIG. 5. Then, the operator (doctor) is informed visually or aurally of the completion of the treatment (Step S12).

According to the present embodiment, as described above, it is concluded that the cauterization process is completed when the phase difference (degrees) is detected by the phase difference detector, and the mode of high-frequency power application can be switched from the constant-power application mode for the cauterization process to the intermittent-power application mode for the desiccation process. Further, the desiccation process for the objective tissue 10 can be concluded to be completed when the predetermined value (e.g., the set value based on the phase difference of 45 degrees) is exceeded by the phase difference (or the changing rate of the phase difference per unit time) that is detected by the phase difference detector.

In the present embodiment, moreover, the switching and completion of the treatment process are determined by the phase difference. Therefore, the influence of a parasitic capacitance generated in the cables connected to the treatment instrument on the switching timing can be eliminated more efficiently than in the case of impedance control, and the high-frequency power can be supplied in consideration of its loss.

FIG. 7 shows a first example of the bipolar treatment instrument used in the present embodiment, and FIG. 8 shows a characteristic of a phase difference generated by the treatment instrument of the first example.

This treatment instrument 31 is configured so that respective one ends of two jaws 32a and 32b that individually serve as electrodes are swingably connected to a sheath 33. In this configuration, the sheath 33 and the jaw 32b are integrally formed straight and fitted with the one end of the jaw 32a for open-close action.

Electrodes 34 and 35 are provided on the inside of the jaws 32a and 32b, respectively. If the objective tissue 10, e.g., a blood vessel, is pinched between the electrodes 34 and 35, it is collapsed more severely on the proximal side (connection side) of the jaws and held in a fan shape, as shown in FIG. 7. If high-frequency power is applied to each of the electrodes 34 and 35 in this state, high-frequency current flows intensively through that part of the objective tissue 10 which is situated on the proximal side of the jaws. Accordingly, adhesion by cauterization advances from the proximal part, and the current gradually increases toward the distal end side opposite from the proximal side. In consequence, the cauterization process starts to be completed from the proximal side first. At this point in time, a phase difference P1 is generated, and the constant-power application mode for the cauterization process is switched over to the intermittent-power application mode for the desiccation process, according to the present embodiment.

Thus, the cauterization (or adhesion) and desiccation (or dehydration) of the objective tissue 10 can be performed more securely by the position control without regard to the holding structure of the treatment instrument.

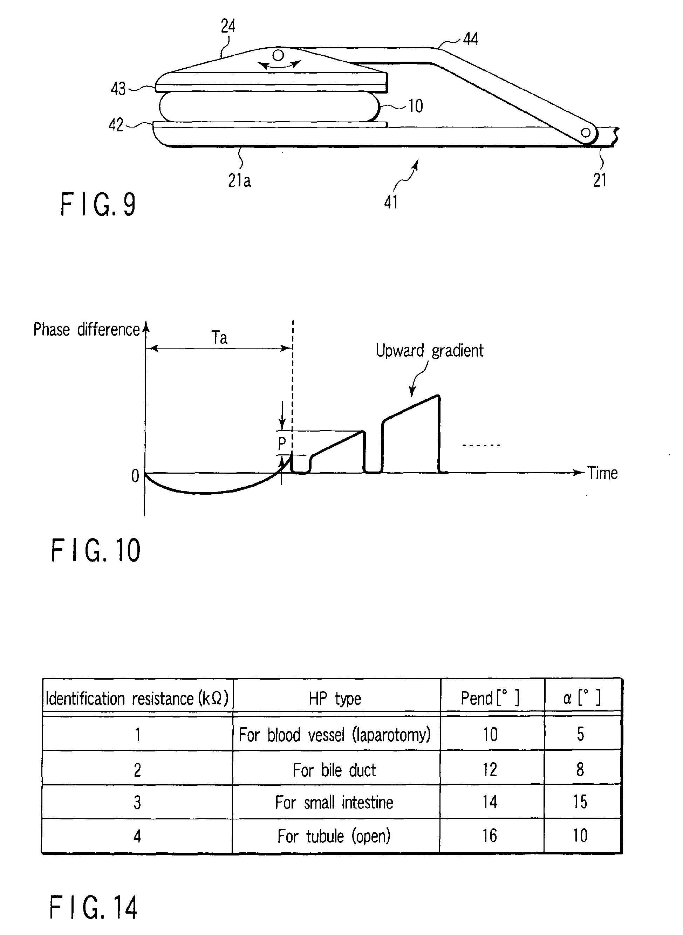

FIG. 9 shows a second example of the bipolar treatment instrument used in the present embodiment, and FIG. 10 shows a characteristic of a phase difference generated by the treatment instrument of the second example.

This treatment instrument 41 is configured so that a first electrode 42 and a second electrode 43 are increased in size in the structure of the treatment instrument 6 shown in FIG. 6, whereby a wider opposite area can be secured. Since the opposite area of the electrodes is wide, a jaw 44 has a bent shape in the middle. Like reference numbers are used to designate other like component parts, and a description of those parts is omitted.

When the treatment instrument 41 holds the objective tissue 10 having a circular cross section, e.g., a blood vessel, the second electrode 43 extends parallel to the first electrode 42 and can evenly crush the tissue 10. Thus, the cauterization and desiccation processes for the tissue 10 can be advanced uniformly.

The treatment instrument 41, compared with the treatment instrument 6 shown in FIG. 6, can more easily hold a large objective tissue taking advantage of its two large-sized electrodes, although a time Ta required for the cauterization process is long, as shown in FIG. 10. In the intermittent-power application mode for the desiccation process, however, the change of a phase difference P obtained has an ascent, so that the changing rate can be recognized with ease. If the phase difference for the completion of the desiccation process is set to, for example, 45 degrees, therefore, the ascent of the phase difference and the set value cross each other, so that the completion of the process can be determined with ease. Thus, the cauterization (or adhesion) and desiccation (or dehydration) of the objective tissue 10 can be performed more securely by the position control without regard to the holding structure of the treatment instrument.

The following is a description of an electric treatment system according to a second embodiment.

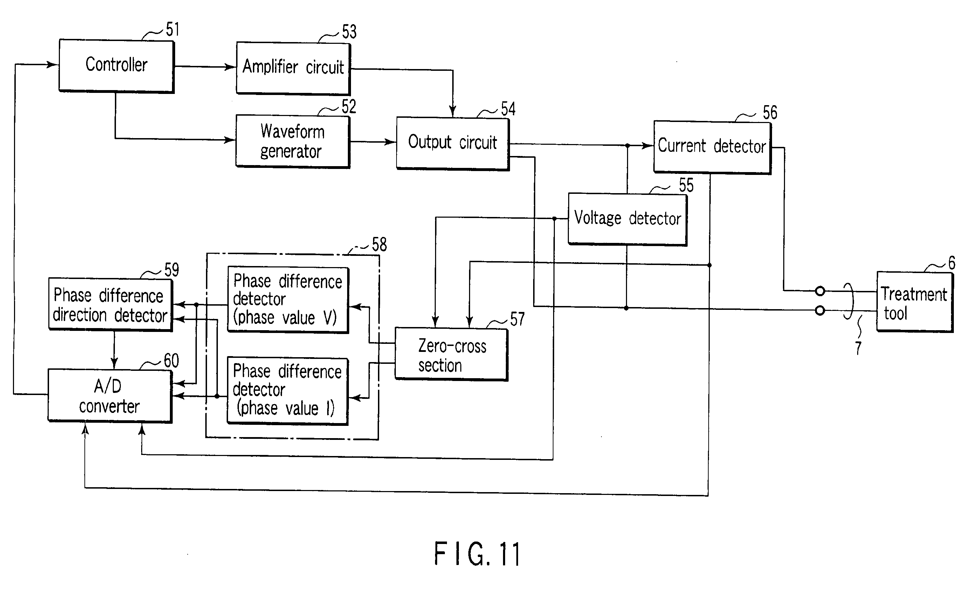

FIG. 11 shows a configuration example of the electric treatment system according to the second embodiment, which will be described in detail. The present embodiment is a system in which a change of the phase direction caused immediately before a phase difference is generated is used as a criterion for process switching from the cauterization process to the desiccation process.

The system of the present embodiment includes a CPU 51, waveform generator 52, amplifier circuit 53, output circuit 54, voltage detector 55, and current detector 56. The CPU 51 serves to control the entire system. The waveform generator 52 generates a waveform of a set mode. The amplifier circuit 53 serves to amplify the waveform from the waveform generator 52. The output circuit 54 is driven by a waveform signal generated by the waveform generator 52. The voltage detector 55 detects the voltage that is supplied from the output circuit 54 to the human body. The current detector 56 detects the current that is supplied from the output circuit 54 to the human body. The system further includes a zero-cross section 57, phase difference detector 58, phase difference direction detector 59, A/D converter 60, impedance calculator 3, and treatment instrument 6. The zero-cross section 57 converts detection signals from the voltage detector 55 and the current detector 56 into phase signals, individually. The phase difference detector 58 detects a difference between the voltage and current phase signals from the zero-cross section 57. The phase difference direction detector 59 detects the direction of the phase difference. The A/D converter 60 digitizes an output signal (analog signal) from the phase difference direction detector 59 and transmits the digitized signal to the CPU 51. The treatment instrument 6 is electrically connected to the output circuit 54 by cables 7. The phase difference detector 58 is composed of a phase difference detector 58a for detecting the phase difference of a voltage V based on a current I and a phase difference detector 58b for detecting the phase difference of the current I based on the voltage V. A system for connection to the treatment instrument 6 by the cables 7 may be configured to use a connector.

In the present embodiment, the direction of the phase difference is detected and used as the criteria in addition to the aforesaid changing rate of the phase difference. The following is a description of the phase difference direction.

The phase difference direction is settled depending on the dominant factor in the total impedance of the output circuit 54. Thus, in order to detect the phase difference direction, it is essential to detect the dominant factor that determines the total impedance. Specifically, the dominant factor can be detected as follows:
a phase direction (1), a factor based on the combined capacitance of the bipolar cables such that the current advances with respect to the voltage, or
a phase direction (2), a factor based on the inductiveness of the bipolar cables such that the voltage advances with respect to the current.

As mentioned before, the phase direction (1) is a factor that ceases to be negligible as the cauterization mainly advances. Thus, the phase direction (1) is an important factor in the observation of the cauterization process for the tissue. Likewise, when the tissue and the treatment instrument 6 are not fully in contact with each other (or in an open state), the phase of the current advances (by 90 degrees) with respect to the voltage, so that the state of contact between the tissue and the electrodes can be discriminated.

With respect to the phase direction (2), moreover, inductive components of the cables are mainly dominant factors. This situation can occur when the resistance is very low with the electrodes and the tissue in contact. Therefore, status discrimination can be performed for the case where the size of the electrodes is very large, for example. Further, the electrodes can be checked for short-circuiting.

Thus, when the phase direction is changed from the combined capacitance state (1) to the inductiveness (2), that is, after the phase difference temporarily deflects from 0 to the negative side (inductive state), as in the section of a phase signal B shown in FIG. 5, the value changes through 0 to the positive side (combined capacitance state). When this change occurs, it is concluded that the cauterization process should be switched over to the desiccation process, whereupon the constant-power application mode is replaced by the intermittent-power application mode.

According to the present embodiment, as described above, the high-frequency power application mode can be switched from the constant-power application mode for the cauterization process to the intermittent-power application mode for the desiccation process, based on the change of the phase difference direction from the inductive state to the combined capacitance state. Further, a minimum value of the phase difference may be detected so that it can be concluded that the treatment process should be switched to the desiccation process when a predetermined amount of phase difference is increased from the minimum value.

In the present embodiment, as in the first embodiment, the switching of the treatment process is determined by the phase difference. Therefore, the influence of an inductive state caused in the cables connected to the treatment instrument on the switching timing can be eliminated more efficiently than in the case of impedance control, and the high-frequency power can be supplied in consideration of its loss.

The following is a description of an electric treatment system according to a third embodiment.

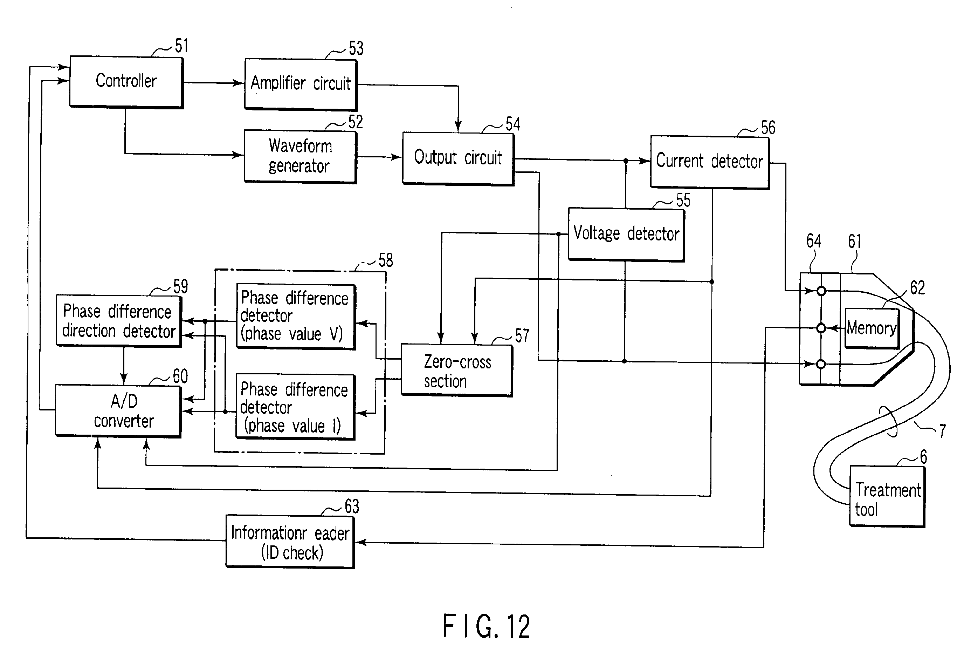

FIG. 12 shows a configuration example of the electric treatment system according to the third embodiment, which will be described in detail. Like reference numbers are used to designate equivalent component parts of the present embodiment and the first embodiment shown in FIG. 1, and a description of those components is omitted.

The present embodiment is configured so that cables 7 that connect a treatment instrument 6 to an output circuit are provided with a removable connector portion 61. A case of the connector portion contains a semiconductor memory unit that is stored with information on the treatment instrument.

The present embodiment is provided with the connector portion 61, a semiconductor memory unit (hereinafter referred to simply as the memory) 62, an information reader 63, and a connector receiver (connection terminal area) 64, besides the system configuration of the foregoing first embodiment. The connector portion 61 includes a plurality of connection terminals and serves for connection with the cables 7. The memory 62 is stored with information on the treatment instrument 6 and the like. The information reader 63 reads the information from the memory 62 and subjects it to an ID check. The connector receiver 64 is removably fitted with the connector portion 61. The memory 62 may be a nonvolatile memory, such as a ROM, PROM, or flash memory, or a rewritable memory that dispenses with storage operation.

The information on the treatment instrument includes a phase difference signal Pend for a value indicative of the completion of the desiccation process, the coefficient (α) or drive conditions proper for each individual objective tissue and forceps, and ID information for specifying the treatment instrument.

The coefficient (α) is a correction value for predetermined normal operation (e.g., treatment time). It is a numerical value that represents any of proper characteristics, such as differences that depend on the objective tissue (blood vessel, small intestine, etc.) to be treated, e.g., the size, composition, water content, etc., of the objective region, the shape of the forceps, or the holding area and heating characteristics of the electrodes. The ID information, which serves to determine the connection of the treatment instrument under control for identification, is not indispensable.

In the present embodiment, the ID information is read from the memory 62 and checked to discriminate the treatment instrument 6 when the connector portion 61 is fitted and electrically connected to the connector receiver 64 on the system body side. As this is done, it is also determined whether or not the information on the treatment instrument 6 is available. Based on authorization by this check, the information reader 63 reads the information on the treatment instrument from the memory 62 and delivers it to a controller 5. The controller 5 sets the information in a preset table (storage region). If there is no checking process for the ID information, the information on the treatment instrument 6 is read out the moment the treatment instrument is connected. Even if an operator (doctor or the like) performs no input operation, therefore, optimum drive conditions and criteria can be set for the treatment instrument merely by mounting it in place.

When this electric treatment system is driven to carry out a treatment by the treatment instrument 6, a decision is made based on coefficients and the like set in accordance with the information on the treatment instrument. Thereupon, the high-frequency power output mode is switched or application is stopped in the cauterization and desiccation processes, as described in connection with the first and second embodiments. It is to be understood that the information setting according to the present embodiment can also be easily applied to the first and second embodiments with the same effect.

The following is a description of a modification of the third embodiment.

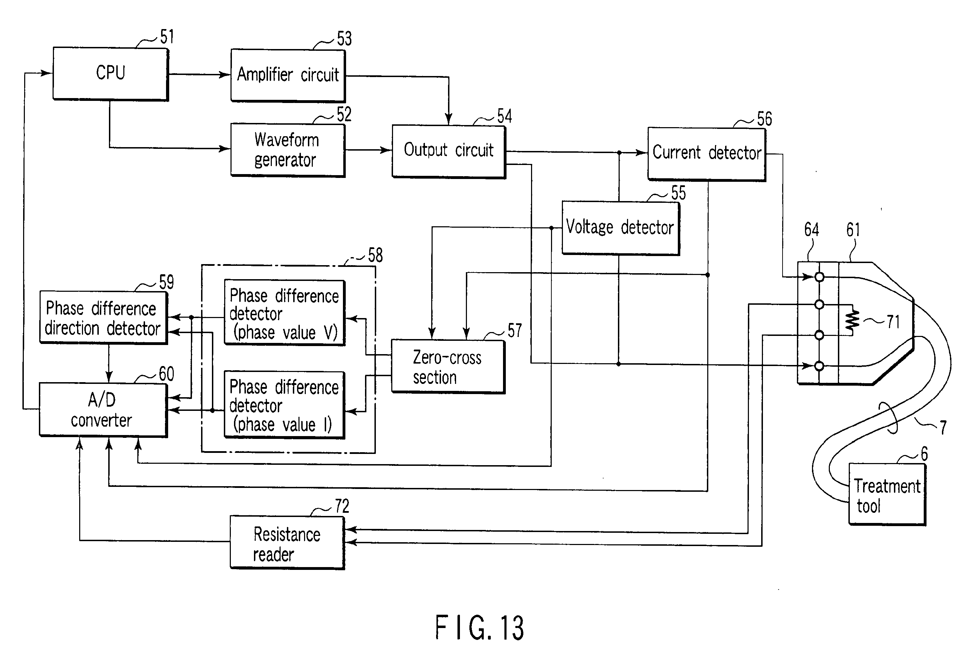

FIG. 13 shows a conceptual configuration of this modification. In the third embodiment described above, the semiconductor memory is provided in the connector portion so that the connector portion (treatment instrument) can secure the information on the treatment instrument. However, this modification is a configuration example in which a connector portion 61 is provided with an index member and a memory unit in a controller 5 is provided with the information (including the coefficient α) on the treatment instrument. Like reference numbers are used to designate equivalent component parts of the present modification and the third embodiment shown in FIG. 12, and a description of those components is omitted.

In the present modification, a resistor is used as the index member. An identification resistor 71 for identifying the treatment instrument is provided in the connector portion 61, while a resistance reader 72 for reading the resistance of the identification resistor 71 is provided on the system body side. The resistance read by the resistance reader 72 is input to the controller 5 after it is digitized by an A/D converter 73.

The controller 5 reads set values from tables that are previously registered depending on the variation of the resistance of the identification resistor 71. FIG. 14 is an example identification table registered in the memory unit of the controller 5.

In this identification table, resistances of a resistor R are distributed in the order of kΩ. Further, each table for each resistance contains the HP (hand-piece) type, phase difference signal Pend (°), and coefficient α (°) of each treatment instrument (e.g., forceps). In the example shown in FIG. 14, "for blood vessel (or laparotomy)" as the HP type, phase difference Pend (10°), and coefficient α (5°) are set individually for terms of an arithmetic expression for a determination process for the controller 5. A metal film resistor or ceramic resistor may be used for the resistor R.

According to this modification, the resistance of the identification resistor that is alternatively mounted in advance is read out merely by connecting the connector portion of the treatment instrument. The items (e.g., phase difference Pend) that are used for the determination specified by the resistance are set in an arithmetic processor of the controller 5. Even if a user (operator or the like) performs no input operation, therefore, optimum drive conditions and criteria can be set for the treatment instrument merely by mounting it in place. Further, the memory can be arranged at a lower cost than the one according to the third embodiment.

Further, the index is not limited to a resistor. If a multi-pin connector is used, the identification table can be set in the same manner as the identification resistance by combining the respective numbers of several pins. Each combination of the pin numbers may, for example, be a combination of each two short-circuited pins, such as pins Nos. 1 and 2, pins Nos. 1 and 3, etc. It is to be understood that the identification may be made based on the presence and positions of the pins.

According to each embodiment of the electric treatment system of the present invention, as described above, satisfactory cauterization and desiccation can be securely performed for objective tissues that are different in some conditions, e.g., composition, size, shape, and water content. By the use of the phase difference control according to the present embodiment, as compared with fixed-time control or impedance control, moreover, the tissue conditions can be grasped more appropriately and the switching and completion of the treatment process can be determined more accurately without the influence of the combined capacitance in the cables.

## Claims

1. An electric treatment system **characterized by** comprising:
a treatment instrument (6) which holds an objective living tissue (10) by means of a holding portion and performs a treatment;
a cable (7) through which high-frequency power is transmitted to the holding portion of the treatment instrument (6);
an output section (13) which is provided with a first output control mode in which the high-frequency power is continuously output to perform the treatment and a second output control mode in which the high-frequency power is intermittently supplied to perform the treatment at intervals based on predetermined output stop times, and alternatively outputs the high-frequency power in the first or second output control mode;
a phase difference detector (4) which detects, as a phase difference signal, a phase difference generated between a high-frequency voltage and a high-frequency current output from the output section (13), based on a combined capacitance between the treatment instrument (6) and the cable (7); and
a controller (5) which controls the entire system and switches the first output control mode over to the second output control mode for the output section (13) when the phase difference signal is detected from the phase difference detector during the treatment by the treatment instrument (6).

2. The electric treatment system according to claim 1, **characterized in that** the controller (5) causes the treatment instrument (6) to perform for the objective living tissue (10) a first treatment based on a cauterization process for adhesion using the high-frequency power in the first output control mode and a second treatment based on a desiccation process for dehydrating a region healed by the adhesion in the second output control mode, and switches the first output control mode over to the second output control mode as the first treatment is replaced by the second treatment when the phase difference is detected from the phase difference detector (4).

3. The electric treatment system according to claim 1, **characterized in that** the controller (5) performs a first treatment based on a cauterization process for adhesion using the high-frequency power in the first output control mode and a second treatment based on a desiccation process for dehydrating a region healed by the adhesion in the second output control mode, and stops the output of the high-frequency power when a predetermined phase difference as a criterion is exceeded by the phase difference detected in the second output control mode.

4. The electric treatment system according to claim 3, **characterized in that** the controller (5) causes a notification portion to issue a notice and, at the same time, cuts off the high-frequency power output, concluding that the second treatment should be terminated, when a predetermined value indicative of 45 degrees is exceeded by an up-to-date phase difference.

5. The electric treatment system according to claim 3, **characterized in that** the criteria of the controller (5) additionally include coefficients given by numerical values which represent proper characteristics, such as the size, composition, and water content of the objective region, the shape of the holding portion of the treatment instrument (6), and the holding area and heating characteristics of electrodes.

6. The electric treatment system according to claim 3, which further comprises a connector portion (61) which removably and electrically connects the cable (7) and the output section (13), a memory unit (62) provided in the connector portion (61) and previously stored with the coefficients, and an information reader (63) which reads the coefficients from the memory unit (62) and delivers the coefficients to the controller (5) through an A/D converter.

7. The electric treatment system according to claim 3, **characterized in that** the controller (5) is loaded with the coefficients in an identification table for each the treatment instrument (6), which further comprises a connector portion (61) which removably and electrically connects the cable (7) and the output section (13), an index member (71) which is provided in the connector portion (61) and has values associated with the identification table, and an information reader (72) which reads out the values of the index member, and wherein the controller reads (72) the coefficients concerned from the identification table based on the read values of the index member (71) and sets the coefficients as the criteria.

8. The electric treatment system according to claim 3, **characterized in that** the index member (71) is composed of a resistor.

9. The electric treatment system according to claim 1, which further comprises a notification portion (8, 9) which notifies the outside with a sound and/or a display.

10. The electric treatment system according to claim 1, **characterized in that** the treatment instrument (6) is of a bipolar type including first and second jaws (21b, 23) of which respective one ends are supported for rocking motion and first and second flat plate-shaped electrodes (22, 25) provided individually on respective opposite surfaces of the jaws (21b, 23), and supplies a high-frequency current between the first and second electrodes (22, 25), thereby incising and coagulating (or cauterizing and desiccating) the objective living tissue (10) held between the electrodes (22, 25).

11. The electric treatment system according to claim 5, **characterized in that** a rockable support member (24) is located on the distal end side of the second jaw (23) of the treatment instrument (6), the support member (24) being provided with the second electrode (25), and the second electrode (25) is formed so as to be swingable with respect to the first electrode (21a).

12. An electric treatment system **characterized by** comprising:
a controller (51) which controls the entire system;
a waveform generator (52) which generates high-frequency waveforms of a plurality of set treatment modes in accordance with instructions from the controller (5);
an amplifier (53) for amplifying the waveforms from the waveform generator (52);
an output section (54) which is supplied with an output voltage from the amplifier and driven by a waveform signal generated by the waveform generator (52);
a voltage detector (55) which detects a voltage of high-frequency power for a treatment supplied from the output section (54);
a current detector (56) which detects a current of the high-frequency power for the treatment supplied from the output section (54);
a zero-cross section (57) which converts detection signals from the voltage detector (55) and the current detector (56) into phase signals, individually, by means of a zero-cross circuit;
a phase difference detector (58) which detects a phase difference signal based on a difference between the voltage and current phase signals from the zero-cross section (57);
a phase difference direction detector (59) which detects the phase difference direction of the phase difference signal obtained by the phase difference detector (58); and
an A/D converter (60) which digitizes an output signal from the phase difference direction detector (58) and transmits the digitized signal to the controller (51),
the treatment modes being switched when the direction of the phase difference before the treatment is changed, based on the direction of the phase difference obtained during the treatment for an objective living tissue (10).

13. A treatment method using an electric treatment system, **characterized by** comprising:
a first treatment based on a cauterization process for adhesion in which continuously output high-frequency power is applied to an objective living tissue (10) held by a treatment instrument; and
a second treatment based on a desiccation process in which high-frequency power is intermittently applied to the held objective living tissue (10) at intervals based on predetermined output stop times, whereby a region healed by the adhesion is dehydrated,
the first treatment being switched over to the second treatment when a phase difference composed of a current component and a voltage component of the high-frequency power applied to the objective living tissue (10) is detected.

14. The treatment method according to claim 13, **characterized in that** the output of the high-frequency power is stopped when a predetermined phase difference as a criterion is exceeded by the phase difference composed of the current and voltage components of the high-frequency power applied to the objective living tissue (10).

15. The treatment method according to claim 13, **characterized in that** the first treatment is switched over to the second treatment when the direction of the phase difference composed of the current and voltage components of the high-frequency power applied to the objective living tissue (10) is shifted from the direction of a phase difference before the treatment.
